Europäisches Patentamt

European Patent Office

Office européen des brevets

(19)

(11) Publication number: **0 066 906
B1**

(12) **EUROPEAN PATENT SPECIFICATION**

(45) Date of publication of patent specification: **15.01.86**

(51) Int. Cl.[4]: **C 07 C 126/08,** C 01 C 1/12,
B 01 D 53/34

(21) Application number: **82200538.5**

(22) Date of filing: **04.05.82**

(54) **Process for the recovery of valuable components from the waste streams obtained in the preparation of urea.**

(30) Priority: **15.05.81 NL 8102391**

(43) Date of publication of application:
**15.12.82 Bulletin 82/50**

(45) Publication of the grant of the patent:
**15.01.86 Bulletin 86/03**

(84) Designated Contracting States:
**AT BE DE FR GB IT NL**

(56) References cited:
**GB-A-1 502 119
GB-A-1 505 445
GB-A-1 528 051
GB-A-1 542 371
GB-A-2 028 311**

**CHEMICAL ABSTRACTS, vol. 93, no. 15, 13th
October 1980, page 311, no. 155164a,
Columbus, Ohio, USA**

**CHEMICAL ABSTRACTS, vol. 89, no. 22, 24th
November 1978, page 105, no. 181820y,
Columbus, Ohio, USA**

(73) Proprietor: **UNIE VAN KUNSTMESTFABRIEKEN
B.V.
Maliebaan 81
NL-3581 CG Utrecht (NL)**

(72) Inventor: **Douwes, Adolphe Marie
Potterstraat 34
NL-6165 AE Geleen (NL)**

(74) Representative: **Roeffen, Wilhelmus Johannes
Maria et al
OCTROOIBUREAU DSM Postbus 9
NL-6160 MA Geleen (NL)**

(56) References cited:
**CHEMICAL ENGINEERING PROCESS, vol. 73,
no. 5, May 1977, pp. 80-84, New York (US);
D.F.BRESS et al: "The startup of two major
urea plants. What was learned during startup
of these two plants is expected to speed the
commissioning of six similar units"**

Courier Press, Leamington Spa, England.

## Description

The invention relates to a process for the recovery of valuable components from the off-gases of a urea granulation system as well as from the liquid waste streams obtained in the preparation of urea.

In the preparation of urea from ammonia and carbon dioxide, a high temperature and corresponding pressure are used to form a urea synthesis solution which still contains a considerable amount of free ammonia and unconverted ammonium carbamate. The carbamate is decomposed in one or more stages into ammonia and carbon dioxide which are for the greater part driven off together with the free ammonia present, and are usually recycled. In the last decomposition stage an aqueous urea solution is obtained which still contains amounts of dissolved ammonia and carbon dioxide, which are subsequently removed by expansion to atmospheric or lower pressure. The aqueous urea solution is concentrated by evaporation and/or crystallization and processed further. Upon evaporation and crystallization a gas mixture is formed which, besides water vapour, contains entrained fine urea droplets, and further ammonia and carbon dioxide. This gas mixture is condensed, as is the gas mixture separated in the expansion of the urea solution after the last decomposition stage. Thus, a so-called process condensate is obtained, part of which is returned into the process for the absorption of the gas mixture discharged from the last decomposition stage. As a rule, the residual part is drained off. The process condensate also includes the water introduced into the process as steam for the operation of the ejectors in the evaporation and/or crystallization section, wash water, rinsing water on the packing glands of the carbamate pumps etc. Per mole of urea one mole of water is formed. This means that in a urea plant with a capacity of 1000 tonnes urea per day, 300 tonnes of water will be formed per day. In addition, depending on the temperature of the cooling water used in the process, approximately 200—315 tonnes of water are introduced per day, so that in total roughly 500—615 tonnes of water are to be discharged per day.

Usually this water still contains 2—9 wt. % $NH_3$, 0.8—6 wt. % $CO_2$ and 0.3—1.5 wt. % urea. These not inconsiderable amounts of feedstock and product are to be recovered, on the one hand in order to minimize the losses of feedstock and product, on the other to prevent them from loading the surface-water into which this waste water would be drained to a degree no longer permitted by the authorities of many countries.

The further processing of the concentrated urea solution or the crystallized urea, that is if urea is to be applied as a fertilizer, as a rule consists of granulation. A much-used method is prilling, a technique which involves the spraying in a prilling tower, in counterflow with a cooling gas, mostly air, of the virtually anhydrous melt that is obtained by evaporation of the aqueous urea solutions or by melting of urea crystals. Another known method for the preparation of urea granules is the spraying of highly concentrated urea solutions or urea melts onto urea seeds kept in fluidized state by air. With these techniques large amounts of air, in which fine liquid and solid urea particles are suspended, are discharged from the granulation system. In a urea plant with a capacity of 1000 tonnes of urea per day the air contained approximately 30—40 kg urea dust per hour if the urea is granulated by prilling. If the fluid-bed granulation technique is applied for granulation, this amount can even be approximately 80 kg urea dust per hour. If this air were to be simply vented, these amounts would cause, apart from a loss of product, a pollution of the air which is no longer permitted by the authorities in many countries, either.

It is already known to remove the greater part of the amounts of ammonia and urea present in the process condensate before the process condensate is drained. To this end, the process condensate can be subjected to a treatment described in Industrial Wastes, September/October 1976 pp. 44—47, in which the process condensate already freed of part of the ammonia and carbon dioxide by desorption at a low pressure is introduced into the bottom of a reaction column at higher pressure and is there heated by means of steam also fed to the bottom, which results in hydrolysis of urea. The solution thus obtained is discharged from the top of the reaction column. Besides a slight amount of non-hydrolysed urea, it contains ammonia and carbon dioxide which, after expansion of the solution to the aforementioned low pressure, are removed in a second desorption column by stripping with steam. The gas mixture thus separated is used as stripping medium in the first desorption stage. The bottom product of the second desorption column is drained off after exchanging heat with the process condensate to be treated. Under practical conditions this waste stream will then still contain approximately 50 ppm $NH_3$ and 50 ppm urea. Even at very long residence times, for which inefficiently large reaction columns would be required, it is not possible to achieve urea contents of less than 20—25 ppm. If the process condensate is treated according to the process described in Dutch patent application 8006477 not yet laid open to public inspection, according to which process the hydrolysis of the urea in the process condensate is carried out in counterflow with an inert gas, preferably steam, in a reaction column, the temperature in the bottom of the column being maintained at 180—230°C and the temperature in the top of the column at 170—220°C, the ammonia and the urea can be removed from the waste stream down to contents of 10 ppm or less. No mention is made in these publications of removal of pollutants from the air stream from the granulation section.

From Chemical Abstracts vol. 89, 1978, no. 181820y it is known to hydrolyze urea present in a dilute aqueous urea-containing solution at a pressure of 8—19 kg/cm² gauge and a tempera-

ture of 175—210°C and to remove the $NH_3$ and $CO_2$ formed in a stripping treatment. In the process described in the British patent specification 1,542,371 the hydrolysis of urea present in the process condensate of the urea process is carried out at a temperature of at least 180°C and a pressure of 18 atmospheres. According to the process described in Chemical Engineering Progress, Vol. 73, (1977) no. 5, pages 80—84, the process condensate of a urea plant is completely evaporated in the high pressure carbamate condensation zone, in which treatment the urea present in the condensate is decomposed into $NH_3$ and $CO_2$ and is carried off together with the steam produced. According to the process of the British patent specification 1,505,445 the water vapour obtained in a urea concentration step may be passed to a suitable mist separator to separate a urea mist therefrom, prior to the condensation of the water vapour, whereafter $NH_3$ and $CO_2$ are removed from the condensate by stripping with low pressure steam. In the above cited publications the removal of pollutants from a granulation step is not mentioned.

In the process described in Chemical Abstracts Vol. 93 (1980) no. 155164a urea mist is removed from the waste gases of urea pelletizing columns by scrubbing the gases and thereafter treating the scrubbed gases in a demister. The hydrolysis of the urea dissolved in the scrubbing solution is not mentioned.

It is also known to lead the air which is discharged in urea granulation processes, and which contains entrained urea particles into the bottom of a washing column, in counterflow with a dilute aqueous urea solution, which has been obtained by condensation of the vapours from the evaporation stage, the urea particles entrained in air stream being washed out and dissolved (see British patent specification 1,528,051). From the bottom of the washing column a urea solution is obtained which is led back to the first evaporation stage. By the heat exchange between the hot off-gases from the granulation system and the wash liquid in the washing column, water evaporates, so that from the top of the washing column a mixture of air and water vapour is discharged and vented.

The process described has the disadvantage that pollutants present in the air used for granulation are washed out in the washing column and end up in the urea solution to be evaporated. Any additives added to the urea melt to be granulated, such as formaldehyde or formaldehyde derivatives, will also end up in the evaporation section via the wash liquid and can impede the process there by formation of foam. If a fairly concentrated urea solution of, say, 20—25 wt. % is to be recycled to the evaporation section, the wash liquid must be circulated over the washing column, yielding the danger of entrainment by the gas stream of drops of the already fairly concentrated urea solution, resulting in a considerably greater loss of urea than when the solution need not be circulated. If in the washing column glass fibres are applied as packing material, the danger of attack on this packing material caused by the high pH of the solution as a result of the presence of ammonia and ammonium cyanate formed by the hydrolysis of urea, is not imaginary.

The invention now offers a process whereby the valuable components which are present in the off-gases of a urea granulation system as well as in the process condensate formed in the preparation of urea can be recovered, while the above-mentioned disadvantages are avoided.

The process according to the invention for the recovery of valuable components from gaseous and liquid waste streams, containing urea and possibly ammonia, that are formed in the preparation of particulate urea products, in which a dilute aqueous urea solution possibly containing ammonia is subjected to a hydrolysis treatment and the ammonia and carbon dioxide thus formed are removed from the solution, is characterized in that the urea is removed from the gaseous waste stream by washing with at least part of the liquid waste stream containing urea, and the solution thus obtained is subjected to the hydrolysis treatment.

Particularly good results are achieved when substantially all ammonia is first removed, from the aqueous urea solution to be used as wash liquid for the gaseous waste stream because then during washing no desorption of ammonia can occur and no valuable starting material can be lost. The removal of the ammonia can for instance be effected by subjecting the liquid waste stream to a stripping treatment with steam or another inert gaseous stripping agent in a pre-desorption column at a pressure of 1—5 bar.

If not the total amount of liquid waste stream is needed for the dissolution of the urea particles from the gaseous effluent, it is advantageous from an economic point of view to conduct the removal of ammonia in two stages. In the first stage the ammonia is removed to such a degree that the subsequent hydrolysis treatment in the hydrolysis column, even in the lower zones where the urea concentration is low, is not impeded. In the second stage the ammonia subsequently is substantially completely removed from the amount needed for the dissolution of the urea particles. The solution obtained in the first stage is fed direct to the hydrolysis treatment, the solution obtained in the second stage is used as wash liquid and solvent for the urea particles in the gaseous waste stream.

The heating and stripping agent to be applied in the hydrolysis column, which can operate at a pressure between 10 and 42 bar, is by preference steam of 12—44 bar. The heat introduced by steam of this pressure is sufficient to hydrolyse urea virtually completely in a short time. Inert gases other than steam are applicable but have to be separated out later, which involves extra cost.

The desorption of the ammonia and carbon dioxide formed in the hydrolysis can be carried out in a desorption column at a pressure of 1—5 bar. The gas mixture obtained in the desorption column and the gas mixture obtained in the top of the hydrolysis column are fed to the pre-desorption column as stripping and heating agents after expansion to the pressure of the desorbed gas mixture. By preference the desorption column and the pre-desorption column are operated at the same pressure.

The gases discharged from the pre-desorption column, a mixture of ammonia, carbon dioxide and water vapour, can be fed, whether or not after complete or partial condensation, to the low-pressure part of the urea synthesis and be processed further there.

With the process according to the invention not only the above-mentioned objections are met, but also, depending on the amount and temperature of the off-gases from the granulation system, 20—30% of the amount of water from the condensate-stream is evaporated. As a result of this, the load of the hydrolysis column is 20—30% less and the amount of high-pressure steam needed in the hydrolysis column and the amount of low-pressure steam needed in the desorption column can be decreased. This advantage amply outweighs the disadvantage of the need for an extra amount of low-pressure steam for complete removal of ammonia from at least part of the liquid waste stream. The extra amount of urea to be hydrolysed is only slightly larger, at most 10%, than the amount of urea to be treated in the known process.

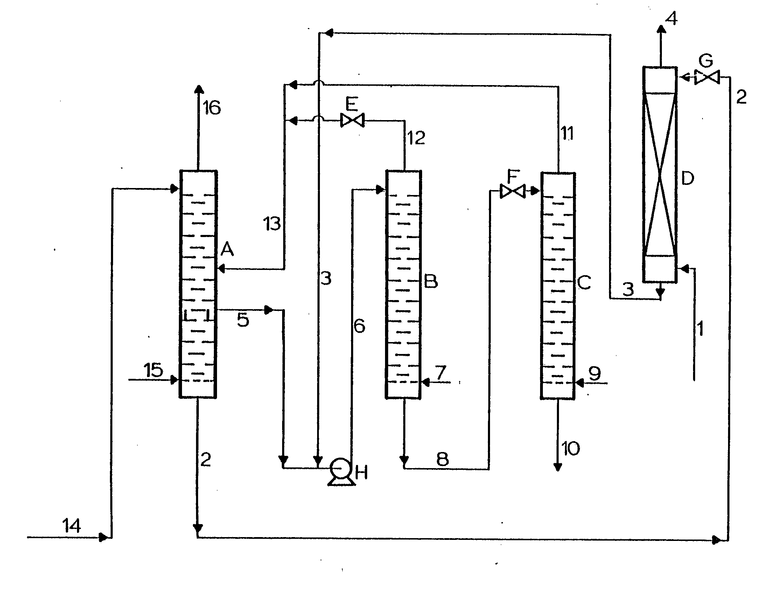

The invention will hereafter be elucidated with reference to the attached drawing. In this, the treatment of the process condensate of the urea preparation and of the off-gases from the urea granulation system is shown diagrammatically.

In the drawing, A represents the pre-desorption column, B the hydrolysis column and C the desorption column. By D the washing column for washing out and dissolving urea particles from the off-gases of the granulation system is indicated. E, F and G refer to expansion valves, while H represents a pump.

In pre-desorption column A the process condensate supplied through 14 is contacted, at a pressure of for instance 1—5 bar, with a mixture of water vapour, ammonia and carbon dioxide supplied through 13. Through 5, part of the process condensate partially free of ammonia and carbon dioxide is led to hydrolysis column B which operates at a pressure of for instance 10—42 bar. Through 15, low-pressure steam is fed to the bottom of pre-desorption column A, resulting in desorption of the ammonia still present in the condensate. From the bottom of column A a practically ammonia-free liquid is discharged through 2, which is subsequently led into washing column D via expansion valve G. To this column dust-laden off-gas from a granulation or prilling section is supplied through 1. The off-gas and water vapour purified here are discharged through 4. The aqueous solution formed in the washing out of the urea particles and any ammonia from the gas stream is discharged from washing column D through 3 and pumped through 6 into hydrolysis column B by pump H, together with the part of the process condensate supplied through 5. High-pressure steam is supplied to this column through 7. The urea present in the supplied solution is here decomposed virtually quantitatively into ammonia and carbon dioxide. The supplied steam not only provides the required amount of heat but also serves as a stripping agent for driving off of part of said ammonia and carbon dioxide. The solution discharged through 8 is expanded to a pressure of for instance 1—5 bar in the expansion valve F and led into desorption column C, to which low-pressure steam is fed through 9 in order to remove the ammonia and carbon dioxide still present from the solution. The gas mixture discharged from the top of desorption column C through 11 is fed into pre-desorption column A through 13, together with the gas mixture discharged from reaction column B, after this latter mixture has been expanded to the pressure of the former gas mixture in expansion valve E. From the top of the column through 16 a gas mixture is obtained in which, in the form of ammonia and carbon dioxide, virtually the total amount of the valuable components present in the off-gas and in the process condensate are contained.

### Example
Using the process described above, the process condensate and the air discharged from the prilling tower, obtained in a urea plant with output of 1000 tonnes per day, were treated.

The amounts are given in kg/hr.

The process condensate, 25,600 kg, contained 5.0 wt. % $NH_3$, 3.15 wt. % $CO_2$ and 1.43 wt. % urea. In the pre-desorption column A at a pressure of 4 bar, the solution was brought in counterflow with 5100 kg gaseous mixture from reaction column B and desorption column C, which consisted of 243 kg $NH_3$, 293 kg $CO_2$ and 4564 kg water vapour and has a temperature of 138°C. Into the bottom of the desorption column, 2800 kg steam with a temperature of 143°C and a pressure of 4.5 bar was led. Through 2, 15,000 kg of a solution containing 188 kg urea, 5 kg $NH_3$, 1 kg $CO_2$ and 14,806 kg water was fed to the top of washing column D. To the bottom of washing column D the air from the prilling tower, 469,300 kg, in which 35 kg $NH_3$, 35 kg urea and 100 kg water vapour, with a temperature of 80°C, was supplied. With the air discharged from washing column D, 5 kg urea, 35 kg $NH_3$ and 8500 kg water vapour were discharged from the system. The solution obtained from the bottom of the washing column through 3, which contained 218 kg urea, 5 kg $NH_3$, 1 kg $CO_2$ and 6406 kg water, together with the solution supplied from the pre-desorption column through 5, consisting of 177 kg urea, 15 kg $NH_3$, 3 kg $CO_2$ and 13,985 kg water, was brought at a pressure of 37 bar by pump H and led into hydrolysis column B. The amount of steam which was led into the bottom of hydrolysis column B was 1000 kg. its temperature was 352°C and its pressure 38 bar. The space velocity was controlled in such a way that the residence time of the liquid was 5—10 minutes. Virtually all of the urea present in the feed was hydrolysed into $NH_3$ and $CO_2$. From the bottom of hydrolysis column B 21,310 kg of a solution consisting of 203 kg $NH_3$, 20 kg $CO_2$ and 21,087 kg water was discharged through 8 which, after lowering of the pressure to 4 bar in F, was treated in desorption column C with 4200 bar steam of 4.5 bar and 143°C. From the bottom of desorption column C 20,190 kg water containing less than 10 ppm urea and less than 10 ppm $NH_3$ was removed from the system. From the top of desorption column C, 4600 kg of a gas mixture composed of 203 kg $NH_3$, 20 kg $CO_2$ and 4377 water vapour, with a temperature 136°C was discharged through 11. From the top of hydrolysis column B 500 kg of a gas mixture consisting of 40 kg $NH_3$, 273 kg $CO_2$ and 187 kg water vapour, with a temperature of 210°C, was discharged through 12. After the pressure of the gas mixture discharged through 12 had been lowered to 4.5 bar in E, the mixture was led through 13 into pre-desorption column A together with the gas mixture supplied through 11. From the top of pre-desorption column A a gas mixture consisting of 1503 kg $NH_3$, 1089 kg $CO_2$ and 1728 kg water vapour was obtained. This gas mixture was subsequently led to the low-pressure part of the urea synthesis.

For the realization of the process according to the invention, approximately 7000 kg low-pressure steam and 1000 kg high-pressure steam are needed per hour. By the heat content of the off-gases from the prilling tower, approximately 8400 kg water are evaporated.

If the process condensate and the off-gases were treated separately, 6800 kg low-pressure steam and 1200 kg high-pressure steam would be needed for the decomposition of urea and the desorption of $NH_3$ and $CO_2$, while for the evaporation of the dilute aqueous solution obtained upon separation of the urea from the off-gases 200 kg low-pressure steam would be needed.

**Claims**

1. Process for the recovery of valuable components from the gaseous and liquid waste streams, containing urea and possibly ammonia, that are formed in the preparation of particulate urea products, in which a dilute aqueous urea solution possibly containing ammonia is subjected to a hydrolysis treatment and the ammonia and carbon dioxide thus formed are removed from the solution, characterized in that the urea is removed from the gaseous waste stream by washing with at least part of the liquid waste stream containing urea and the solution thus obtained is subjected to the hydrolysis treatment.

2. Process according to claim 1, characterized in that first substantially all ammonia is removed from the dilute aqueous urea solution to be used as wash liquid from the gaseous waste stream.

3. Process according to claim 2, characterized in that the removal of ammonia is carried out in two stages, the solution obtained in the first stage is directly subjected to the hydrolysis treatment and the solution obtained in the second stage is used as wash liquid for the gaseous waste stream.

**Patentansprüche**

1. Verfahren zur Wiedergewinnung verwertbarer Komponenten aus den gasförmigen und flüssigen, Harnstoff und gegebenenfalls Ammoniak enthaltenden Abfallproduktströmen die bei der Herstellung von teilchenförmigen Harnstoffprodukten gebildet werden, wobei eine verdünnte, gegebenenfalls Ammoniak enthaltende wässerige Harnstofflösung einer Hydrolysebehandlung unterworfen wird un das dabei gebildete Ammoniak und Kohlendioxid aus der Lösung entfernt werden, dadurch gekennzeichnet, daß der Harnstoff aus dem gasförmigen Abfallproduktstrom durch Auswaschen mit wenigstens einem Teil des flüssigen, Harnstoff enthaltenden Abfallproduktstromes entfernt und die so erhaltene Lösung der Hydrolysebehandlung unterworfen wird.

2. Verfahren nach Anspruch 1, dadurch gekennzeichnet, daß zuerst im wesentlichen das ganze Ammoniak aus der als Waschflüssigkeit für den gasförmigen Abfallproduktstrom zu verwendenden verdünnten, wässerigen Harnstofflösung entfernt wird.

3. Verfahren nach Anspruch 2, dadurch gekennzeichnet, daß die Entfernung des Ammoniaks in zwei Stufen durchgeführt wird, wobei die in der ersten Stufe erhaltene Lösung direkt der Hydrolysebehandlung unterworfen und die in der zweiten Stufe erhaltene Lösung als Waschflüssigkeit für den gasförmigen Abfallproduktstrom verwendet wird.

**Revendications**

1. Procédé de récupération de substances utiles à partir d'effluents liquides et gazeux, contenant de l'urée et éventuellement de l'ammoniac, qui sont formés au cours de la préparation de produits particulaires d'urée, dans lequel une solution aqueuse diluée d'urée pouvant contenir de l'urée est soumise à un traitement d'hydrolyse et l'ammoniac et le dioxyde de carbone ainsi formés sont séparés de la solution, caractérisé en ce que l'urée est séparé de l'effluent gazeux par lavage avec au moins une partie de l'effluent liquide contenant l'urée, et la solution ainsi obtenue est soumise au traitement d'hydrolyse.

2. Procédé selon la revendication 1, caractérisé en ce que sensiblement tout l'ammoniac est d'abord séparé de la solution aqueuse diluée d'urée à utiliser comme liquide de lavage pour l'effluent gazeux.

3. Procédé selon la revendication 2, caractérisé en ce que la séparation de l'ammoniac est réalisée en deux étapes, la solution obtenue dans la première étape est directement soumise au traitement d'hydrolyse et la solution obtenue dans la seconde étape est utilisée comme liquide de lavage pour l'effluent gazeux.